# EUROPÄISCHE PATENTSCHRIFT

(11) **EP 2 448 897 B1**
(45) Veröffentlichungstag und Bekanntmachung des Hinweises auf die Patenterteilung: **14.10.2015**
(21) Anmeldenummer: 10734027.5
(22) Anmeldetag: 12.06.2010
(51) Int. Cl.: C07C 29/80, C07C 41/09, C07C 41/42, C07C 31/04, C07C 43/04, B01D 3/14

(54) **VERFAHREN UND ANLAGE ZUR HERSTELLUNG VON METHANOL UND DIMETHYLETHER**
METHOD AND SYSTEM FOR PRODUCING METHANOL AND DIMETHYL ETHER
PROCÉDÉ ET INSTALLATION POUR LA FABRICATION DE MÉTHANOL ET D ÉTHER DIMÉTHYLIQUE

(30) Priorität: 03.07.2009 DE 102009031636
(43) Veröffentlichungstag der Anmeldung: 09.05.2012
(73) Patentinhaber: Air Liquide Global E&C Solutions Germany GmbH, 60439 Frankfurt am Main (DE)
(72) Erfinder: AHLERS, Bernd, 63128 Dietzenbach (DE); LIEBNER, Waldemar, 61440 Oberursel (DE)
(74) Vertreter: Keil & Schaafhausen Patent- und Rechtsanwälte
(86) Internationale Anmeldenummer: PCT/EP2010/003549
(87) Internationale Veröffentlichungsnummer: WO 2011/000470

(56) Entgegenhaltungen:
- WO-A1-96/28408
- JP-A- 2004 161 673
- US-A- 4 744 869
- DATABASE WPI Week 200936 Thomson Scientific, London, GB; AN 2009-H89867 XP002598503 -& CN 101 412 665 A ((HEDD-I) HE D) 22. April 2009 (2009-04-22)

## Beschreibung

Die vorliegende Erfindung betrifft ein Verfahren zur Herstellung, von aufgereinigtem Methanol (MeOH) und/oder Dimethylether (DME) aus Rohmethanol sowie eine Anlage zur Durchführung dieses Verfahrens.

Dimethylether (C₂H₆O, DME) ist ein Ether mit zwei Methylgruppen als organischen Resten. Hochreiner DME findet breite Anwendung als Treibgas. Aufgrund einer Cetanzahl von 55 bis 60 lässt sich Dimethylether auch im Dieselmotor als Ersatz für Dieselkraftstoff verwenden. Er gilt als Biokraftstoff sofern er aus Biomasse hergestellt ist und soll langfristig Flüssiggas ablösen. Daher hat DME in den vergangenen Jahren erheblich an Bedeutung gewonnen.

Die Herstellung von Dimethylether kann durch säurekatalysierte Kondensation von Methanol unter Abspaltung von Wasser erfolgen:

₂CH₃OH → CH₃-O-CH₃ + H₂O

Dieses Verfahren zur Herstellung von Dimethylether aus Methanols wird u. a. in "Ullmann's Encyclopedia of Chemistry, Sixth Edition, 1998 Electronic Release" beschrieben. Dabei wird Methanoldampf über einen mit Dehydratisierungskatalysator gefüllten Reaktor geleitet, an den sich eine zweistufige Destillation anschließt. In der ersten Destillationsstufe wird Dimethylether als Kopfprodukt gewonnen, während im Sumpfprodukt vor allem nicht umgesetztes Methanol und Produktwasser enthalten ist. Dieses Sumpfprodukt wird in der zweiten Destillationsstufe aufgetrennt, wobei Methanol als Kopfprodukt abgezogen wird. Das als Sumpfprodukt erhaltene Wasser wird verworfen oder in einem anderen Prozess verwendet.

Aus praktischen Gründen wird eine Dimethyletherherstellung im technischen Maßstab oft direkt an ein Verfahren zur Methanolgewinnung angeschlossen.

Die US 4 744 869 A beschreibt ein Verfahren zur Reinigung von Methanol. Dazu wird das Methanol zuerst in eine Vorreinigungsstufe und dann in eine Endreinigungsstufe geführt.

Die WO 96/28408 A1 lehrt hierzu einen Prozess für die Produktion und Gewinnung von Dimethylether durch Dehydratisierung von Methanol. Der Energieverbrauch in der Destillation soll dadurch verringert werden, dass das aus der ersten Destillationsstufe erhaltene Sumpfprodukt über einen Stripper geführt wird, in dem eine grobe Trennung von Wasser und Methanol erfolgt. Der vorgereinigte Methanolstrom wird dann in die Methanolzuleitung eingespeist und in eine Destillationskolonne überführt, die dem Dimethylreaktor vorgeschaltet ist. Dadurch wird der gesamte in den Reaktor eingespeiste Methanolstrom aufgearbeitet und die Aufreinigung des Sumpfproduktes nach dem DME-Reaktor verkürzt.

Ähnlich versucht auch die JP 2004/161673 A, die Trennung des Produktes DME von unumgesetztem Methanol mit einer Methanolrückführung zu verbinden. Dazu wird das gesamte Rohmethanol in einer Destillationskolonne gereinigt und anschließend in einem DME-Reaktor zu Dimethylether umgesetzt. Das in der nachgeschalteten Destillation anfallende Sumpfprodukt wird zurück in den Rohmethanolstrom und mit diesem in die destillative Vorreinigung geleitet.

Die CN101412665A offenbart schließlich ein Verfahren zur simultanen Herstellung von Methanol und Dimethylether. Das rohe Methanol wird über die Vorreinigung geführt und so aufgereinigt. Anschließend wird es einer Verdampfungsstufe zugeführt, in der es verdampft wird und anschließend gasförmig in den Dimethyletherreaktor geführt werden kann. Im Reaktor wird das aufgereinigte und verdampfte Methanol an einem Katalysator zu Dimethylether umgesetzt, wodurch auch Wasser entsteht. Das Rohprodukt, welches den Dimethyletherreaktor verlässt, wird in eine Rektifikationskolonne gespeist. Das hier zurückgewonnene, nicht umgesetzte Methanol wird anschließend in einer Methanolrückgewinnungskolonne aufgereinigt, so dass es wieder dem Verdampfer zugeführt werden kann. Überdies werden Verunreinigungen aus dem Sumpf des Verdampfers abgezogen und ebenfalls dieser Methanolrückgewinnungskolonne zugeführt, so dass eventuell enthaltenes Methanol nicht der Wertschöpfungskette entzogen wird, sondern ebenfalls recycelt werden kann.

Den beschriebenen Anlagen ist gemeinsam, dass sie nicht für eine simultane Methanol- und Dimethyletherproduktion ausgelegt sind, sondern das gesamte Rohmethanol in Dimethylether umgewandelt wird. Dies führt jedoch dazu, dass die Produktion der Anlage nicht flexibel auf die durch den Markt gegebenen Anforderungen hinsichtlich Produktionskapazitäten reagieren kann.

Aufgabe der vorliegenden Erfindung ist es daher, ein Verfahren bereitzustellen, mit dem parallel sowohl aufgereinigtes Methanol als auch Dimethylether erzeugt werden kann, wobei die entstehenden Mengen beider Produkte flexibel einstellbar sein sollen.

Diese Aufgabe wird erfindungsgemäß mit den Merkmalen des Anspruchs 1 dadurch gelöst, dass der Rohmethanolstrom in mindestens einer Vorreinigungsstufe aufgearbeitet und das so vorgereinigte Methanol aufgeteilt und einer Methanol-Endreinigung sowie einer Dimethylether-Produktion zugeführt wird. Nicht umgesetztes Methanol aus der Dimethylether-Produktion wird nach dem Reaktor aus mindestens einer Reinigungsstufe abgezogen und der Methanol-Endreinigung zugeführt. Dies erlaubt es, den vorgereingten Methanolstroms variabel auf die Methanol-Endreinigung und die Dimethylether-Produktion aufzuteilen, wodurch das Gesamtverfahren flexibilisiert und an die jeweils aktuellen Bedürfnisse des Marktes angepasst werden kann. Zudem können durch die Kombination der Destillationen Anlagenteile eingespart werden.

Da mit dem erfindungsgemäßen Verfahren eine flexible Umschaltung zwischen einer 100 %igen Methanol-Produktion und einer 100 %igen DME-produktion ermöglicht wird, wird unter dem der Methanolendreinigung bzw. der DME-Produktion zugeführten Teilstrom im Sinne der vorliegenden Erfindung jeweils ein beliebiger Anteil zwischen 0 und 100 % verstanden.

Vorteilhafterweise erfolgt die Vorreinigung und/oder die Endreinigung des Methanols destillativ, da so anders als in anderen Trennverfahren keine weiteren Stoffe wie Adsorbentien oder Lösemittel in den Prozess eingebracht werden müssen.

Bei einer besonders bevorzugten Ausführungsform wird bei der Endreinigung die Methanoldestillation in einer Kolonne mit erhöhtem Druck und/oder einer Kolonne unter Atmosphärendruck durchgeführt. Dadurch kann Einfluss auf die jeweiligen Siedepunkte des zu trennenden Gemisches genommen werden. Bei Hintereinanderschaltung von zwei Kolonnen, nämlich Druck- und Atmosphärenkolonne, kann der Reinheitsgrad durch die thermische Trennung an die Zusammensetzung des Stoffgemisches angepasst werden.

Bevorzugt ist weiterhin eine Ausgestaltung des Verfahrens, bei dem zumindest Teile des in der Dimethylether-Reinigungsstufe abgezogenen Methanolstroms in die Druckkolonne und/oder in die atmosphärische Kolonne geführt werden. Dadurch kann diese Methanolmenge ebenfalls wertsteigernd verarbeitet werden.

Vorzugsweise erfolgt auch die Aufreinigung des Dimethylethers destillativ, um die Vorteile eines thermischen Trennverfahrens, insbesondere die Abwesenheit von Zusatzstoffen, zu nutzen.

Ein Teilstrom des stabilisierten Methanolstroms wird erfindungsgemäß dazu genutzt, nicht kondensierten Dimethylether nach der DME-Kolonne zurückzugewinnen. Vorzugsweise wird das stabilisierte Feedmethanol als Waschmittel zur Abtrennung des DME von den Leichtsiedern eingesetzt.

In einer weiteren bevorzugten Ausgestaltung des Verfahrens wird mindestens ein Teilstrom des Methanols nach einer der Endreinigungsstufen dem für die Umsetzung zu Dimethylether bestimmten vorgereinigten Methanolstrom zugeführt. Diese Zuführung kann sowohl vor als auch nach einer weiteren Aufreinigungsstufe erfolgen und ermöglicht so auch bei geringem Methanolbedarf eine ausreichende Auslastung der Methanol-Endreinigung.

Weiterhin ist eine zusätzliche Vorreinigung des Rohmethanols möglich, indem die Aufteilung des vorgereinigten Methanolstroms auf die Methanol-Endreinigung und die Dimethylether-Produktion erst nach einer weiteren Reinigungsstufe, z. B. der Druckkolonne, vorgenommen wird. Dadurch besitzt der vorgereinigte Methanolstrom einen höheren Reinheitsgrad, was die Dehydratisierungsreaktion und die nachgeschalteten Reinigungsstufen entlastet.

Gemäß einer bevorzugten Ausgestaltung der Erfindung, bei der die Integration der Anlagenteile erweitert wird, wird das gasförmige Kopfprodukt der Druckkolonne dem DME-Reaktor zugeführt, wobei der Methanolstrom vor Eintritt in den DME-Reaktor noch zusätzlich verdichtet werden kann.

Die Erfindung umfasst weiterhin auch eine Anlage zur Herstellung von aufgereinigtem Methanol und Dimethylether aus Rohmethanol, die zur Durchführung des oben beschriebenen Verfahrens geeignet ist und die Merkmale des Anspruchs 10 aufweist. Diese Anlage beinhaltet mindestens eine Vorreinigungsstufe zur Aufarbeitung des Rohmethanols, nach der über ein geeignetes Leitungssystem mindestens eine Endreinigungsstufe für eine weitere Aufreinigung des Methanols und mindestens ein Dimethylreaktor zur Umsetzung des vorgereinigten Methanols zu Dimethylether parallel so miteinander verschaltet sind, dass beide Verfahren simultan durchführbar sind. Dadurch wird eine Flexibilisierung der Anlage hinsichtlich der Produktionskapazitäten von aufgereinigtem Methanol und Dimethylether erreicht.

Dem Dimethylether-Reaktor ist eine Reinigungsstufe nachgeschaltet, in der nicht umgesetztes Methanol von dem Ether abgetrennt wird. Dieses Methanol wird dann durch eine Leitung wenigstens einer der Einrichtungen zur Methanol-Endreinigung zugeführt.

In Weiterbildung der Erfindung besteht sowohl die Methanol-Vorreinigung als auch die Methanol-Endreinigung aus mindestens einer Destillationskolonne. Dies ermöglicht eine Aufarbeitung des Methanols ohne die Einbringung weiterer Stoffe in die Anlage.

Bevorzugt ist zudem eine Ausgestaltung der Mefhanol-Endreinigung, die zwei Kolonnen aufweist, von denen eine unter erhöhtem Druck und eine unter Atmosphärendruck betrieben wird, um eine optimierte Trennung von Methanol und Wasser zu gewährleisten. Zur Minimierung des Energieverbrauchs können die beiden Kolonnen energetisch gekoppelt werden. Dabei wird vorzugsweise ein Wärmetauscher so verwendet, dass er gleichzeitig als Kondensator der Druckkolonne und als Verdampfer der atmosphärischen Kolonne eingesetzt wird, wodurch die Wärme des Kopfproduktes der einen Kolonne energetisch vorteilhaft auf das Sumpfprodukt der anderen Kolonne übertragen wird. Die Verwendung eines zweiten Verdampfers für die atmosphärische Kolonne ist jedoch ebenso möglich.

Von dem Kopf der DME-Reinigungskolonne führt erfindungsgemäß eine Leitung zu einer Reinigungsvorrichtung, um nicht kondensiertes DME zurückzugewinnen. Eine Ausgestaltung dieser Reinigungsstufe als Waschkolonne führt zu einem vergleichsweise geringen Energiebedarf in diesem Aufarbeitungsschritt, allerdings sind in gleicher Weise auch andere Trennvorrichtungen denkbar.

Durch eine Zufuhrleitung, die mindestens eine der Vorrichtungen zur Methanol-Endreinigung mit dem Dimethylether-Reaktor verbindet, kann zusätzlich Methanol zu DME verarbeitet werden, während die Methanol-Endreinigung weiterhin oberhalb ihrer Mindestlast betrieben wird.

Weiterbildungen, Vorteile und Anwendungsmöglichkeiten der Erfindung ergeben sich auch aus der nachfolgenden Beschreibung von Ausführungsbeispielen und der Zeichnung. Dabei bilden alle beschriebenen Merkmale für sich oder in beliebiger Kombination den Gegenstand der Erfindung, auch unabhängig von ihrer Zusammenfassung in den Ansprüchen oder deren Rückbeziehung.

Es zeigen:
- Fig. 1: schematisch eine Anlage zur Herstellung von aufgereinigtem Methanol (MeOH) und/oder Dimethylether (DME) gemäß einer ersten Ausführungsform,
- Fig. 2: schematisch eine Anlage zur Herstellung von aufgereinigtem Methanol (MeOH) und/oder Dimethylether (DME) gemäß einer zweiten Ausführungsform,
- Fig. 3: schematisch eine Anlage zur Herstellung von aufgereinigtem Methanol (MeOH) und/oder Dimethylether (DME) gemäß einer dritten Ausführungform und
- Fig. 4: schematisch eine Anlage zur Herstellung von aufgereinigtem Methanol (MeOH) und/oder Dimethylether (DME) gemäß einer vierten Ausführungsform.

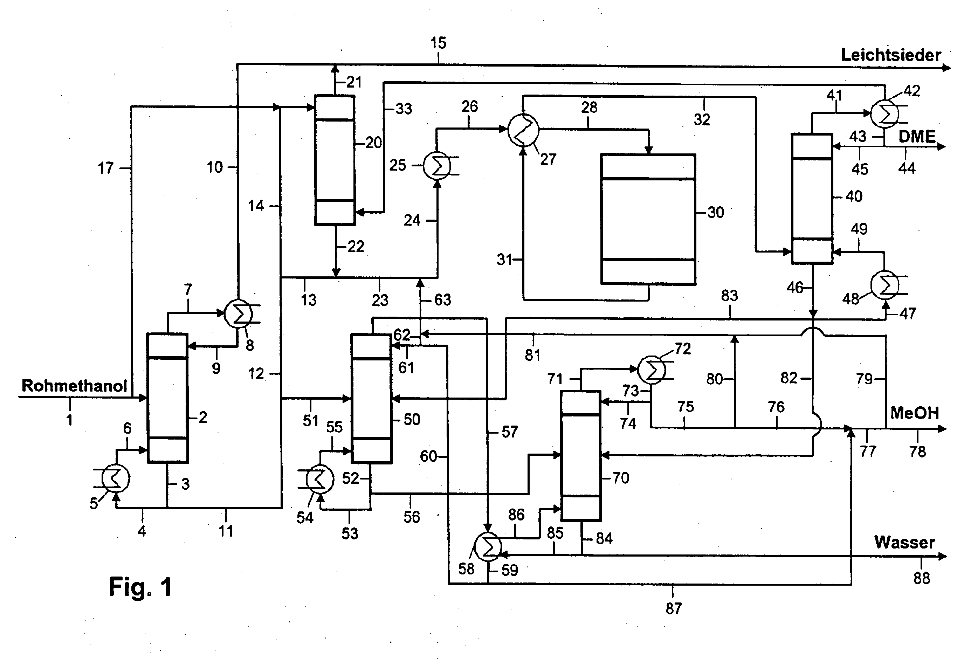

In Figur 1 ist schematisch eine Anlage zur Durchführung des erfindungsgemäßen Verfahrens dargestellt. Über Leitung 1 wird Rohmethanol (Methanol mit Restwassergehalt) direkt aus der Produktion oder auch aus geeigneten Lagervorrichtungen zugeführt und in einer Destillationskolonne 2 vorgereinigt und stabilisiert, aus der über Leitung 7 der Dampf im Kolonnenkopf abgezogen und zumindest teilweise in einem Kondensator 8 wieder verflüssigt wird. Das Kondensat wird über Leitung 9 in die Kolonne 2 rückgeführt, während das aus Leichtsiedern bestehende Destillat über Leitung 10 aus dem Prozess ausgeschleust wird. Das Sumpfprodukt wird über Leitung 3 aus der Kolonne 2 abgezogen und in einen Produktstrom und einen Rücklaufstrom aufgeteilt. Der Rücklaufstrom wird über Leitung 4 in einen Verdampfer 5 und von dort über Leitung 6 zurück in die Kolonne 2 geführt, während das solcherart vorgereinigte Methanol als Produktstrom der Kolonne 2 über Leitung 11 abgeführt wird.

Teile (0 bis 100 %) des stabilisierten Methanolstroms treten über Leitung 51 in den Abschnitt der Anlage ein, in dem die Methanol-Endreinigung durchgeführt wird. Von dem verbleibenden vorgereinigten Methanol in Leitung 12 (0 bis 100 %) kann ein Teil über Leitung 13 direkt der DME-Produktion zugeführt werden, während der Rest des Methanolstroms über Leitung 14 zunächst als Waschmittel in einen Wäscher 20 geleitet wird. Alternativ kann ein Teilstrom des Rohmethanols über Leitung 17 direkt dem Wäscher (Scrubber) 20 zugeführt werden und dort als Waschmittel zur Verfügung stehen.

Über diesen Wäscher 20 wird aus dem über Kopf der DME-Kolonne 40 abgezogenen Leichtsiederstrom mit Hilfe eines Teilstroms des stabilisierten Feedmethanols aus Strom 14 und/oder 17 Dimethylether zurückgewonnen, welcher wegen der Light-Ends im Kopf der DME-Kolonne 40 nicht kondensiert und über Strom 33 an diese Stelle zurückgeführt wurde. Mittels Leitung 21 werden die Leichtsieder aus dem Wäscher 20 mit dem Strom aus Leitung 10 vereinigt und über Leitung 15 abgezogen. Der Methanolstrom wird mit dem zurückgewonnenen DME über Leitung 22 aus dem Wäscher 20 abgezogen und mit dem Methanol aus Leitung 13 vereinigt. Über Leitungen 23 und 24 gelangt das Methanol in einen Wärmetauscher 25, aus dem es, vorzugsweise dampfförmig, durch Leitung 26 in einen Kreuzwärmetauscher 27 gelangt.

Der in dem Kreuzwärmetauscher 27 weiter erhitzte Strom wird über Leitung 28 in einen Dimethylether-Reaktor 30 eingebracht. In diesem DME-Reaktor 30 wird Methanol kontinuierlich vorzugsweise durch säurekatalytische Kondensation, bevorzugt bei einer Temperatur im Bereich von 150 bis 450 °C und einem Druck von 1 bis 40 bar, zu Dimethylether umgesetzt. Das so entstandene Produktgemisch wird mit Leitung 31 in den Kreuzwärmetauscher 27 eingebracht, wo es abgekühlt wird und gleichzeitig den Eduktstrom aus Leitung 26 erhitzt. Durch Leitung 32 wird der abgekühlte Produktstrom zur Destillationskolonne 40 transportiert. Über Kopf werden aus der Kolonne 40 in Leitung 41 die gasförmigen Komponenten abgezogen, die dann zumindest teilweise in einem Kondensator 42 auskondensiert werden. Die flüssigen Anteile des Stroms aus Leitung 43 werden über Leitung 45 zurück in die Kolonne 40 eingespeist, während durch Leitung 44 das Produkt Dimethylether (DME) abgeführt wird. Das wasserreiche Sumpfprodukt der Kolonne 40 wird über Leitung 46 abgezogen und kann sowohl über Leitung 47 in den Verdampfer 48 und von dort durch Leitung 49 zurück in die Kolonne 40 als auch über die Leitungen 82, 83 der Methanol-Endreinigung zugeführt werden, wobei es wegen des erhöhten Wasseranteils vorzugsweise zur atmosphärischen Kolonne 70 geleitet wird. Die Aufteilung auf die beiden Kolonnen 50, 70 erfolgt so, dass die Qualität des verbleibenden Methanolprodukts erhalten bleibt. Dadurch kann auch das nicht umgesetzte Methanol aus der DME-Produktion einer wertsteigernden Aufarbeitung unterzogen werden.

Parallel zu der DME-Produktion wird derjenige Anteil des vorgereinigten und stabilisierten Methanols, der mit Leitung 51 abgezweigt wurde, direkt der Mm'" thanol-Endreinigung zugeführt. Durch Leitung 51 wird Methanol dazu in eine Druckkolonne 50 eingebracht, in der das Methanol unter erhöhtem Druck von 5 bis 20 bar, vorzugsweise 8 bar, destilliert wird. Das Kopfprodukt dieser Destillationsstufe 50 wird über Leitung 57 in den Wärmetauscher 58 und Leitung 59 geleitet. Der Rücklauf aus dem Wärmetauscher 58 wird über Leitung 60 zumindest teilweise mittels Leitung 61 in die Kolonne 50 rückgeführt, während über Leitung 62 und Leitung 63 der Strom auch anteilig der Zuleitung 24 des DME-Reaktors 30 zugeführt werden kann. Das auf MeOH-Produktqualität aufgereinigte Destillat wird über Leitung 87 ausgeschleust. Das im Vergleich zum Rohmethanol mit Wasser angereicherte Sumpfprodukt der Kolonne 50 wird über Leitung 52 abgezogen, aufgetrennt und ein Teilstrom durch Leitung 53 in den Verdampfer 54 geführt, von wo aus dieser Teilstrom mittels Leitung 55 zurück in die Druckkolonne 50 gelangt.

Der verbleibende Methanolstrom wird durch Leitung 56 in eine zweite Kolonne 70 eingebracht, die unter atmosphärischem Druck betrieben wird. Deren Sumpfprodukt gelangt durch Leitung 84 zumindest teilweise in Leitung 85 und von dort in den Wärmetauscher 58 und wird über Leitung 86 teilverdampft wieder in die Kolonne 70 zurückgeführt. Der verbleibende Anteil des im wesentlichen aus Wasser bestehenden Sumpfproduktes wird über Leitung 88 aus dem Prozess ausgeschleust. Es kann in die Abwasserreinigung geleitet oder beispielsweise in der Synthesegaserzeugung wiederverwendet werden. Das Kopfprodukt der Kolonne 70 wird hingegen mittels Leitung 71 in den Wärmetauscher 72 eingebracht. Der aus diesem Wärmetauscher 72 in Leitung 73 austretende Strom wird so aufgeteilt, dass der Rücklauf über Leitung 74 wieder in die atmosphärische Kolonne 70 gelangt, während das Destillat über Leitung 75 abgezogen wird. Falls es für besondere Betriebsbedingungen erforderlich ist, kann die atmosphärische Kolonne 70 mit einem zweiten Aufkocher ausgerüstet werden, der bei Betrieb mit DME-Produktion zugeschaltet werden kann.

Aus dieser Leitung 75 kann über die Leitungen 80, 81 und 63 zusätzlich gereinigtes Methanol in die Zufuhrleitung 24 des DME-Reaktors 30 eingebracht werden. Über die Leitung 76 kann das gereinigte Methanol aus der Kolonne 70 auch mit dem aus Druckkolonne 50 in Leitung 87 abgezogenen Methanol vereinigt und über die Leitungen 77, 78 aus der Anlage abgezogen werden. Die Variabilität des Verfahrens wird zusätzlich dadurch gesteigert, dass ein Teilstrom des Methanols über Leitung 79 in die zur DME-Produktion führende Leitung 81 eingebracht wird. Somit kann sowohl aus der Druckkolonne 50 als auch aus der atmosphärisch betriebenen Kolonne 70 aufgereinigtes Methanol in den Feedstrom des DME-Reaktors 30 eingespeist werden.

Wenn das methanolreiche Kopfprodukt der atmosphärischen Kolonne 70 vollständig zur DME-Produktion zurückgeleitet wird, kann die atmosphärische Kolonne 70 mit reduziertem Rücklauf betrieben werden, da für die Rückführung zum DME-Reaktor 30 keine Methanolproduktqualität erforderlich ist.

Mit der erfindungsgemäßen Anlage kann der Anteil des zu produzierenden Methanols und Dimethylethers variabel gestaltet werden.

Wenn die Anlage nur Methanol produzieren soll, wird das gesamte stabilisierte Methanol aus der Vorlaufkolonne 2 zur Methanoldestillation 50, 70 weitergeleitet. Sowohl in der Druckkolonne 50 als auch der atmosphärischen Kolonne 70 wird spezifikationsgerechtes Methanolprodukt erzeugt, während in diesem Betriebszustand kein DME produziert wird.

Wenn die Anlage DME und Methanol produzieren soll, wird ein Teilstrom des stabilisierten Methanols aus der Vorreinigung 2 zum DME-Reaktor 30 geleitet. Der verbleibende Reststrom wird zur Methanoldestillation 50, 70 geleitet. Da der Gleichgewichtsumsatz der Reaktion vom Methanol zu DME bei ca. 80 % liegt, muss für eine DME-Produktionskapazität von 80 % des stabilisierten Methanols das gesamte stabilisierte Methanol zum DME-Reaktor 30 geleitet werden. Bis zu dieser DME-Produktkapazität kann der DME-Reaktor 30 als once through-Reaktor betrieben werden, wobei das im DME-Reaktor 30 nicht umgesetzte Methanol und das Prozesswasser, also das Sumpfprodukt der DME-Kolonne 40, über die Leitung 46 und 82 bzw. 83 zur Methanoldestillation 50, 70 geleitet und dort zu Methanolprodukt destilliert wird. Der über Leitung 46 abgezogene Strom aus dem Sumpf der DME-Kolonne 40 ist dabei im Wesentlichen frei von Leichtsiedern, was durch eine entsprechende Bodenzahl im Abtriebsteil der Kolonne 40 und/oder die entsprechende Rückflussrate der Kolonne 40 eingestellt werden kann. Sollten andere Verunreinigungen dieses Stroms dazu führen, dass die Spezifikation des Methanols (vorzugsweise Grade AA) nicht eingehalten werden kann, muss das Methanol über Leitung 82 und 83 ganz oder teilweise zur Vorlaufkolonne 2 zurückgeleitet werden. Zur Optimierung der Energie-Effizienz der Anlage kann es sinnvoll sein, einen Restanteil DME im Strom der Leitung 46 zuzulassen. In diesem Fall ist jedoch keine Rückführung aus den Leitungen 82 und 83 möglich, sondern der gesamte Strom muss zur Vorlaufkolonne 2 geleitet werden, wodurch der darin enthaltene DME-Anteil verlorengeht.

Wenn die Anlage ausschließlich DME produzieren soll, dann wird aus dem Sumpfprodukt der DME-Kolonne 40 in der Metharioldestillation das Prozesswasser abgetrennt und das nicht umgesetzte Methanol über die Leitung 81 zum DME-Reaktor 30 zurückgeführt.

Bei einer speziellen Ausführungsform der Erfindung wird in der Druckkolonne 50 Methanolprodukt erzeugt, während das Kopfprodukt der atmosphärischen Kolonne 70 zum DME-Reaktor 30 zurückgeführt und das Sumpfprodukt der DME-Kolonne 40 ausschließlich über die Leitungen 46, 82 zur atmosphärischen Kolonne 70 geleitet wird. Dadurch wird vermieden, dass in der Druckkolonne 50 ein Stoffaustausch mit Stoffströmen der DME-Produktion stattfindet. Dementsprechend wird die Druckkolonne 50 mit demselben Einsatzmaterial betrieben wie bei reiner Methanolproduktion.

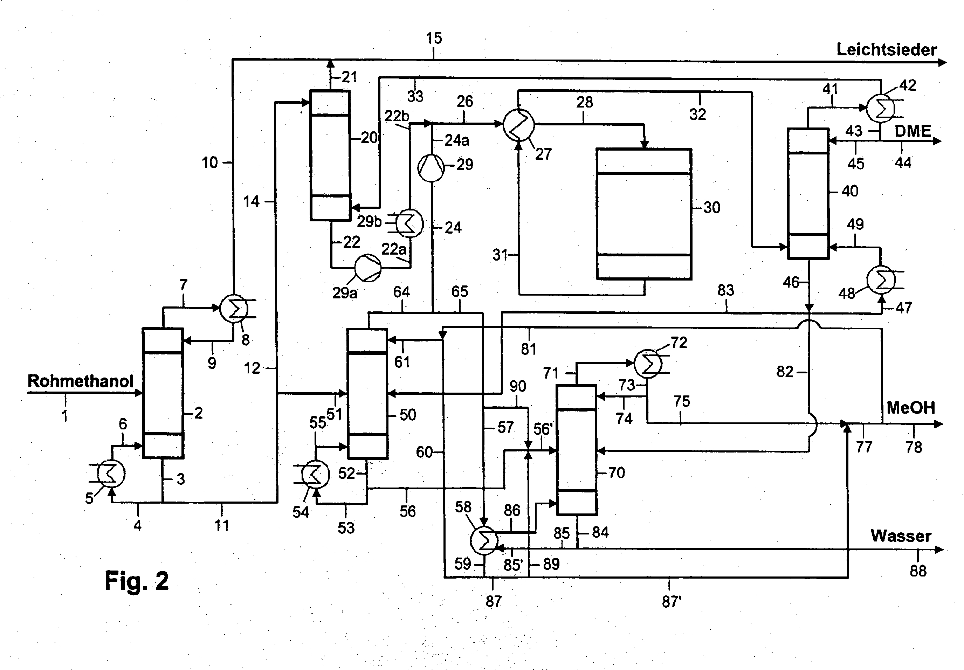

In Figur 2 ist schematisch eine zweite Ausführungsform einer Anlage zur Durchführung des erfindungsgemäßen Verfahrens dargestellt, bei der die Integration beider Anlagenteile erweitert wird. Grundsätzlich setzt sich diese Ausführungsform aus den gleichen Verfahrensstufen zusammen wie die in Figur 1 gezeigte Anlage. Insoweit wird auf die obige Beschreibung verwiesen und im Wesentlichen nur noch auf die bestehenden Unterschiede eingegangen.

Auch bei der zweiten Ausführungsform wird ein Teil des in der Vorreinigung 2 stabilisierten Methanols als Waschmittel in den Wäscher 20 zur Rückgewinnung von nicht kondensierten Dimethylether aus dem Kopfprodukt der DME-Kolonne 40 eingebracht. Das DME enthaltende Sumpfprodukt wird mittels einer Pumpe 29a über Reaktordruck gepumpt, in einem Verdampfer 29b separat verdampft und gemeinsam mit Strom 24a als Strom 26 im Wärmetauscher 27 überhitzt und dem DME-Reaktor 30 zugeführt. So kann in Kolonne 50 und Kolonne 70 Grade AA Methanol ohne DME produziert werden kann.

Wenn nicht das gesamte Kopfprodukt der Druckkolonne 50 in die DME-Produktion geleitet wird, kann der Reststrom über Leitung 65 als Produkt abgezogen und/oder wenigstens teilweise über die Leitung 90 gasförmig und/oder nach Kondensation im Wärmetauscher 58 über die Leitungen 59, 87 und 89 flüssig in den Zulauf 56' der atmosphärischen Kolonne 70 eingespeist werden. Der aus dem Sumpf der atmosphärischen Kolonne 70 mittels Leitung 84 abgezogene wasserreiche Methanolstrom kann über die Leitungen 85 und 86 in den Sumpf der Kolonne 70 rückgeführt werden.

Eine Rückführung des nicht umgesetzten Methanols aus dem Sumpfprodukt der DME-Destillationskolonne 40 erfolgt über Leitung 46, von der sowohl zumindest teilweise Methanol über Leitung 83 in die Druckkolonne 50 der Methanol-Endreinigung als auch zumindest teilweise Methanol über Leitung 82 in die atmosphärische Kolonne 70 der Methanol-Endreinigung eingebracht werden kann.

Durch diese Ausgestaltung des Verfahrens wird das Zusammenspiel zwischen Methanol-Endreinigung und DME-Produktion zusätzlich erweitert, wobei die Kapazität jeder Komponente jeweils so ausgelegt ist, dass sowohl DME als auch aufgereinigtes MeOH zu 100 % erzeugt werden kann.

Das stabilisierte Methanol wird vollständig in der Druckkolonne 50 verdampft und somit die Wasserabtrennung gänzlich in diesen Anlagenteil verlagert. Zudem kann das verdampfte Methanol für die DME-Produktion bereits vollständig zum Reaktor 30 geleitet werden, was allerdings bedingt, dass der Betriebsdruck der Druckkolonne 50 über dem des DME-Reaktors 30 liegen muss. Vorzugsweise ist der Druck der Druckkolonne 50 so gewählt, dass über eine einstufige Kompression des gasförmigen Kopfproduktes in Kompressor 29 der erforderliche Druck zur Weiterleitung zum DME-Reaktor 30 erreicht wird. Dies ist besonders dann erfüllt, wenn die Druckkolonne bei etwa 8 bar abs und der Reaktor 30 bei etwa 16 bar abs. betrieben werden. Durch den Einbau des Verdichters 29 können auch schon bestehende Methanolanlagen für die DME-Produktion umgerüstet werden. Der Verdampfer 25 nach Figur 1 kann entfallen, da die Verdampfung bereits in der Kolonne 50 erfolgt.

Bei einem Betrieb der Anlage, der zu einer 100%-igen DME-Herstellung führt, kann auch das in der zweiten, atmosphärisch betriebenen Kolonne 70 über Kopf gewonnene Methanol Wasser enthalten und wird deshalb zur weiteren DME Produktion über Leitung 81 als Rückfluss der Druckkolonne 50 aufgegeben. Da die Konzentrationen der jeweiligen Kopfprodukte und damit die Rücklaufverhältnisse in den beiden Kolonnen 50 und 70 flexibel sind, können sie so eingestellt werden, dass ihre Verdampfer-Kondensator-Kopplung erhalten bleibt. Zudem kann der atmosphärischen Kolonne ein zweiter Verdampfer zugefügt werden.

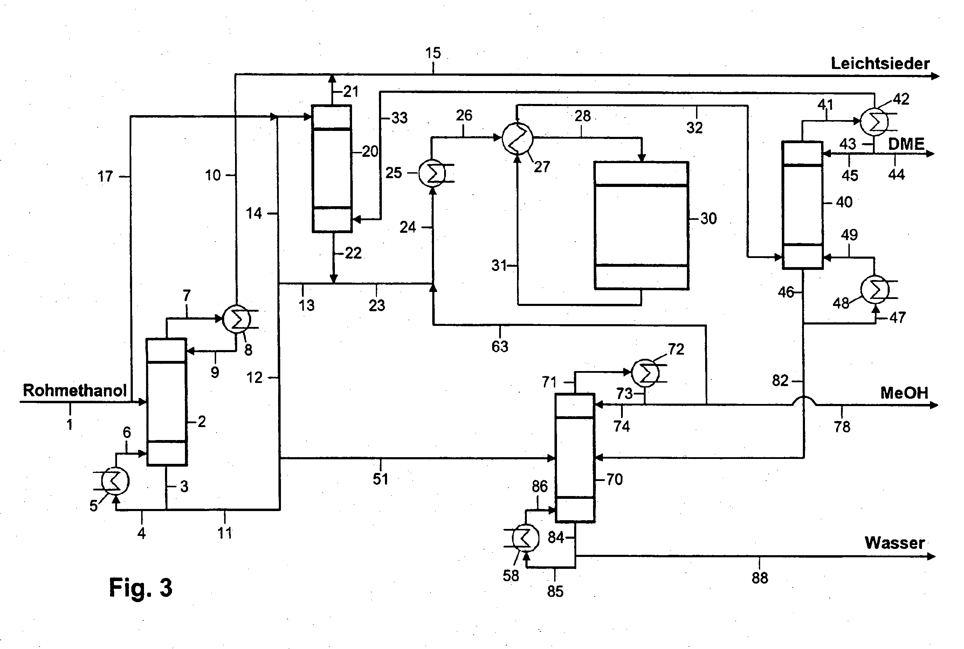

Figur 3 zeigt als dritte Ausführungsform eine Anlage mit einer niedrigeren Methanolkapazität. Neben den schon in Figur 1 ausführlich erläuterten Einheiten zur Methanolvorreinigung 2, der Rückgewinnung von DME in Wäscher 20, dem DME-Reaktor 30 und der Reinigung des so erzeugten Dimethylethers in mindestens einer entsprechenden Vorrichtung 40 liegt deshalb für die Methanol-Endreinigung nur eine, vorzugsweise atmosphärisch betriebene Kolonne 70 vor. Bei der DME-Produktion nicht umgesetztes Methanol kann über die Leitung 82 in die atmosphärische Kolonne 70 eingebracht werden. Das Kopfprodukt der Methanoldestillation wird als Produkt abgezogen oder gelangt über Leitung 63 zurück in den Leitungsverband der DME-Produktion.

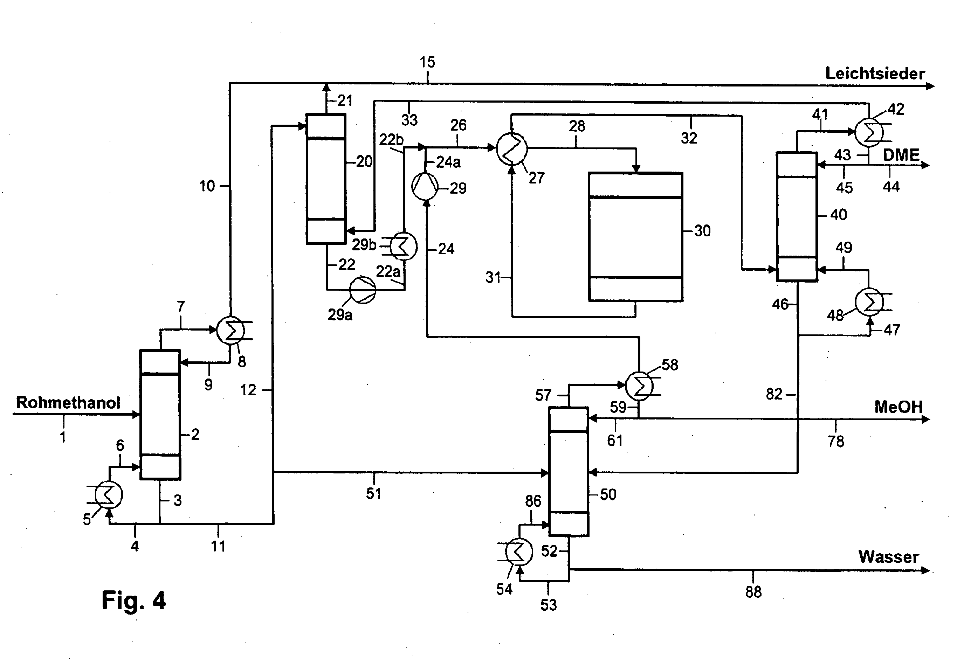

Analog zu der in Figur 2 dargestellten Verfahrensvariante ergibt sich die in Figur 4 dargestellte Anlagenkonfiguration. Hierbei wird die Methanol-Endreinigung ebenfalls mit nur einer Kolonne 50 durchgeführt, allerdings wird diese unter erhöhtem Druck betrieben. Dies ermöglicht eine direkte Einspeisung des aus der Druckkolonne 50 abgezogenen gasförmigen Methanols in den DME-Reaktor 30, wobei optional wieder die Verwendung eines zusätzlichen Verdichters 29 möglich ist. Eine Rückführung des nicht umgesetzten Methanols erfolgt aus der DME-Kolonne 40 über die Leitungen 46 und 82 in die Kolonne 50. Durch Leitung 22 ist es in dieser Ausgestaltung wie auch in Figur 2 möglich, das im Wäscher 20 anfallende Sumpfprodukt über Reaktordruck zu pumpen, separat zu verdampfen und dem DME-Reaktor 30 zuzuführen. Wie schon bei der Ausführungsform gemäß Figur 2 wird auch hier die Verdampfung des Methanols zur Produktion von Dimethylether in die Kolonne 50 verlagert. Bei gleichzeitiger Produktion von Methanol und DME muss hierbei das Kopfprodukt der Druckkolonne 50 Methanolproduktqualität haben. Dementsprechend wird die DME-Produktion mit Reinmethanol betrieben.

### Bezugszeichenliste

- 1: Leitung
- 2: MeOH-Vorreinigung
- 3,4: Leitung
- 5: Wärmetauscher
- 6,7: Leitung
- 8: Wärmetauscher
- 9-15: Leitung
- 17: Leitung
- 20: Wäscher
- 21-24: Leitung
- 25: Wärmetauscher
- 26: Leitung
- 27: Kreuzwärmetauscher
- 28: Leitung
- 29: Verdichter
- 29a: Pumpe
- 29b: Verdampfer
- 30: DME-Reaktor
- 31-33: Leitung
- 40: DME-Reinigungseinrichtung
- 41: Leitung
- 42: Wärmetauscher
- 43-47: Leitung
- 48: Wärmetauscher
- 49: Leitung
- 50: Druckkolonne
- 51-53: Leitung
- 54: Wärmetauscher
- 55-57: Leitung
- 58: Wärmetauscher
- 59-65: Leitung
- 70: atmosphärische Kolonne
- 71: Leitung
- 72: Wärmetauscher
- 73-77: Leitung
- 78: Wärmetauscher
- 79-92: Leitung

## Patentansprüche

1. Verfahren zur Herstellung von aufgereinigtem Methanol und Dimethylether aus Rohmethanol,
wobei das Rohmethanol in mindestens einer Vorreinigungsstufe aufgearbeitet wird,
wobei ein erster Teilstrom des vorgereinigten Methanols einer Methanolendreinigung zugeführt wird und
wobei ein zweiter Teilstrom des vorgereinigten Methanols einem Reaktor zugeführt und wenigstens teilweise zu Dimethylether umgesetzt und der gewonnene Dimethylether in mindestens einer Reinigungsstufe aufgereinigt wird, und
wobei aus der Dimethylether-Reinigungsstufe nicht umgesetztes Methanol abgezogen und wenigstens teilweise der Methanolendreinigung zugeführt wird.

2. Verfahren nach Anspruch 1, **dadurch gekennzeichnet, dass** die Vorreinigung des Methanols, die Endreinigung des Methanols und/ oder die Aufreinigung des Dimethylethers destillativ erfolgt.

3. Verfahren nach Anspruch 1 oder 2, **dadurch gekennzeichnet, dass** die Endreinigung des Methanols in einer ersten Kolonne, die unter erhöhtem Druck betrieben wird, und/oder einer zweiten Kolonne, die unter Atmosphärendruck betrieben wird, durchgeführt wird.

4. Verfahren nach Anspruch 3, **dadurch gekennzeichnet, dass** der aus der Dimethylether-Reinigungsstufe abgezogene Methanolstrom wenigstens teilweise der Druckkolonne und/oder der atmosphärischen Kolonne zugeführt wird.

5. Verfahren nach einem der vorhergehenden Ansprüche, **dadurch gekennzeichnet, dass** aus dem gasförmigen Kopfprodukt der Dimethyletherreinigung bevorzugt durch eine Wäsche mit einem methanolreichen Waschmittel Dimethylether zurückgewonnen wird.

6. Verfahren nach einem der vorhergehenden Ansprüche, **dadurch gekennzeichnet, dass** nach mindestens einer der Endreinigungsstufen wenigstens ein Teilstrom des Methanols dem für die Umsetzung zu Dimethylether bestimmten Methanolstrom zugeführt wird.

7. Verfahren nach einem der Ansprüche 3 bis 6, **dadurch gekennzeichnet, dass** die Aufteilung des Methanolstroms auf die Methanolendreinigung und die Dimethyletherherstellung nach der Druckkolonne erfolgt.

8. Verfahren nach einem der Ansprüche 3 bis 7, **dadurch gekennzeichnet, dass** das gasförmige Kopfprodukt der Druckkolorine wenigstens teilweise dem Dimethylether-Reaktor zugeführt wird.

9. Verfahren nach Anspruch 8, **dadurch gekennzeichnet, dass** das Kopfprodukt der Druckkolonne vor Einführung in den Dimethylether-Reaktor verdichtet wird.

10. Anlage zur Herstellung von aufgereinigtem Methanol und/oder Dimethylether aus Rohmethanol, insbesondere zur Durchführung eines Verfahrens nach einem der vorhergehenden Ansprüche, mit mindestens einer Vorreinigungsstufe (2) des Rohmethanols, mindestens einer Endreinigungsstufe (50 bzw. 70) zur Endreinigung des Methanols und mindestens einem Dimethylether-Reaktor (30) zur Umsetzung des Methanols in Dimethylether, wobei die Zuleitungen (11, 51) zur Methanol-Endreinigung (50, 70) und zum Dimethylether-Reaktor (30) beide mit der Methanol-Vorreinigung (2) verbunden sind, wobei eine dem DME-Reaktor (30) nachgeschaltete Dimethylether-Reinigungsstufe (40) über eine Methanol-Rückführleitung (46) mit wenigstens einer Methanol-Endreinigungsstufe (50 bzw. 70) verbunden ist.

11. Anlage nach Anspruch 10, **dadurch gekennzeichnet, dass** die Methanolvorreinigung und/oder die Methanol-Endreinigung wenigstens eine Destillationskolonne (2, 50, 70) aufweist.

12. Anlage nach Anspruch 11, **dadurch gekennzeichnet, dass** die Methanol-Endreinigung zwei Destillationskolonnen (50 und 70) umfasst, von denen eine unter erhöhtem Druck und eine unter Atmosphärendruck betrieben wird.

13. Anlage nach einem der Ansprüche 10 bis 12, **dadurch gekennzeichnet, dass** eine Rückführungsleitung (33) aus dem Kopf der DME-Reinigungskolonne (40) an mindestens eine Reinigungsvorrichtung, bevorzugt eine Waschkolonne (20), angeschlossen ist.

14. Anlage nach einem der Ansprüche 10 bis 13, **dadurch gekennzeichnet, dass** mindestens eine Stufe (50, 70) der Methanol-Endreinigung mit der Zuflussleitung (26) des Dimethylether-Reaktor (30) verbunden ist.

## Claims

1. A process for producing purified methanol and dimethyl ether from crude methanol,
wherein the crude methanol is processed in at least one prepurification stage,
wherein a first partial stream of the prepurified methanol is supplied to a final methanol purification, and
wherein a second partial stream of the prepurified methanol is supplied to a reactor and at least partly converted to dimethyl ether, and the dimethyl ether recovered is purified in at least one purification stage, and
wherein non-reacted methanol is withdrawn from the dimethyl ether purification stage and at least partly supplied to the final methanol purification.

2. The process according to claim 1, **characterized in that** the prepurification of the methanol, the final purification of the methanol and/or the purification of the dimethyl ether is effected by distillation.

3. The process according to claim 1 or 2, **characterized in that** the final purification of the methanol is performed in a first column, which is operated under elevated pressure, and/or in a second column which is operated under atmospheric pressure.

4. The process according to claim 3, **characterized in that** the methanol stream withdrawn from the dimethyl ether purification stage is at least partly supplied to the pressure column and/or the atmospheric column.

5. The process according to any of the preceding claims, **characterized in that** from the gaseous top product of the dimethyl ether purification, preferably by washing with a washing agent rich in methanol, dimethyl ether is recovered.

6. The process according to any of the preceding claims, **characterized in that** after at least one of the final purification stages at least one partial stream of the methanol is supplied to the methanol stream intended for conversion to dimethyl ether.

7. The process according to any of claims 3 to 6, **characterized in that** splitting up the methanol stream onto the final purification of methanol and the production of dimethyl ether is effected after the pressure column.

8. The process according to any of claims 3 to 7, **characterized in that** the gaseous top product of the pressure column is at least partly supplied to the dimethyl ether reactor.

9. The process according to claim 8, **characterized in that** the top product of the pressure column is condensed before introduction into the dimethyl ether reactor.

10. A plant for producing purified methanol and/or dimethyl ether from crude methanol, in particular for performing a process according to any of the preceding claims, comprising at least one prepurification stage (2) of the crude methanol, at least one final purification stage (50, 70) for the final purification of the methanol, and at least one dimethyl ether reactor (30) for converting the methanol to dimethyl ether, wherein the feed conduits (11, 51) to the final methanol purification (50, 70) and to the dimethyl ether reactor (30) both are connected with the methanol prepurification (2) wherein a dimethyl ether purification stage (40) provided downstream of the DME reactor (30) is connected with at least one final methanol purification stage (50, 70) via a methanol return conduit (46).

11. The plant according to claim 10, **characterized in that** the methanol prepurification and/or the final methanol purification includes at least one distillation column (2, 50, 70).

12. The plant according to claim 11, **characterized in that** the final methanol purification comprises two distillation columns (50 and 70), one of which is operated under elevated pressure and one under atmospheric pressure.

13. The plant according to any of claims 10 to 12, **characterized in that** a return conduit (33) from the top of the DME purification column (40) is connected to at least one purification device, preferably to a washing column (20).

14. The plant according to any of claims 10 to 13, **characterized in that** at least one stage (50, 70) of the final methanol purification is connected with the supply conduit (26) of the dimethyl ether reactor (30).

## Revendications

1. Procédé de production de méthanol purifié et d'éther diméthylique à partir de méthanol brut,
ledit méthanol brut étant traité dans au moins un étage de pré-purification,
un premier débit partiel du méthanol pré-purifié étant introduit dans un dispositif de purification définitive de méthanol, et
un deuxième débit partiel du méthanol pré-purifié étant introduit dans un réacteur pour le faire réagir au moins partiellement de manière à obtenir de l'éther diméthylique, et l'éther diméthylique ainsi obtenu étant purifié dans au moins un étage de purification, et
le méthanol qui n'a pas réagi étant récupéré à l'issue de l'étage de purification d'éther diméthylique pour l'introduire au moins partiellement dans le dispositif de purification définitive de méthanol.

2. Procédé selon la revendication 1, **caractérisé en ce que** la pré-purification du méthanol, la purification définitive du méthanol et/ou la purification de l'éther diméthylique sont mises en oeuvre par distillation.

3. Procédé selon les revendications 1 ou 2, **caractérisé en ce que** la purification définitive du méthanol se déroule dans une première colonne mise en oeuvre à une pression élevée et/ou dans une deuxième colonne mise en oeuvre à pression atmosphérique.

4. Procédé selon la revendication 3, **caractérisé en ce que** le débit de méthanol récupéré à l'issue de l'étage de purification d'éther diméthylique est au moins partiellement introduit dans la colonne sous pression et/ou dans la colonne atmosphérique.

5. Procédé selon l'une des revendications précédentes, **caractérisé en ce qu'**à partir du produit gazeux obtenu en tête du dispositif de purification d'éther diméthylique, on récupère de l'éther diméthylique, de préférence par lavage avec un agent de lavage riche en méthanol.

6. Procédé selon l'une des revendications précédentes, **caractérisé en ce qu'**au moins un débit partiel du méthanol, obtenu en aval d'au moins un des étages de purification définitive, est introduit dans le débit de méthanol destiné à réagir de manière à obtenir de l'éther diméthylique.

7. Procédé selon l'une des revendications 3 à 6, **caractérisé en ce que** la répartition du débit de méthanol entre le dispositif de purification définitive de méthanol et le dispositif de production d'éther diméthylique est mise en oeuvre en aval de la colonne sous pression.

8. Procédé selon l'une des revendications 3 à 7, **caractérisé en ce que** le produit gazeux obtenu en tête de la colonne sous pression est au moins partiellement introduit dans le réacteur d'éther diméthylique.

9. Procédé selon la revendication 8, **caractérisé en ce que** le produit obtenu en tête de la colonne sous pression est comprimé avant d'être introduit dans le réacteur d'éther diméthylique.

10. Installation de production de méthanol et/ou éther diméthylique purifié(s) à partir de méthanol brut, destinée notamment à la mise en oeuvre d'un procédé selon l'une des revendications précédentes, laquelle comporte au moins un étage de pré-purification (2) du méthanol brut, au moins un étage de purification définitive (50 ou 70) destiné à définitivement purifier le méthanol, et au moins un réacteur d'éther diméthylique (30) destiné à faire réagir le méthanol de manière à obtenir de l'éther diméthylique, les ligne d'introduction (11, 51) menant vers le dispositif de purification définitive de méthanol (50, 70) et vers le réacteur d'éther méthylique (30) étant toutes les deux reliées au dispositif de pré-purification de méthanol (2), un étage de purification de éther diméthylique (40), placé en aval du réacteur de DME (30), étant relié à au moins un étage de purification définitive de méthanol (50 ou 70) par une ligne de recyclage de méthanol (46).

11. Installation selon la.revendication 10, **caractérisée en ce que** le dispositif de pré-purification de méthanol et/ou le dispositif de purification définitive de méthanol comporte(nt) au moins une colonne de distillation (2, 50, 70).

12. Installation selon la revendication 11, **caractérisé en ce que** le dispositif de purification définitive de méthanol comprend deux colonnes de distillation (50 et 70) dont une est mise en ouvre à une pression élevée et une est mise en oeuvre à pression atmosphérique.

13. Installation selon l'une des revendications 10 à 12, **caractérisée en ce qu'**une ligne de recyclage (33), partant de la tête de la colonne de purification de DME (40), est branchée sur au moins un dispositif de purification, de préférence sur une colonne de lavage (20).

14. Installation selon l'une des revendications 10 à 13, **caractérisée en ce qu'**au moins un étage (50, 70) du dispositif de purification définitive de méthanol est relié à la ligne d'introduction (26) du réacteur d'éther diméthylique (30).
